# EUROPÄISCHE PATENTSCHRIFT

(11) **EP 1 024 086 B1**
(45) Veröffentlichungstag und Bekanntmachung des Hinweises auf die Patenterteilung: **11.08.2004**
(21) Anmeldenummer: 00100386.2
(22) Anmeldetag: 08.01.2000
(51) Int. Cl.: B65B 55/08, A61L 2/10

(54) **Vorrichtung zum Entkeimen von Packstoffbahnen**
Apparatus for sterilising webs of packaging material
Dispositif pour stériliser des bandes en matériau d'emballage

(30) Priorität: 28.01.1999 DE 19903259
(43) Veröffentlichungstag der Anmeldung: 02.08.2000
(73) Patentinhaber: Hassia Verpackungsmaschinen GmbH, D-63691 Ranstadt (DE)
(72) Erfinder: Kurth, Gunter, 63691 Ranstadt (DE); Sabotka, Ingo, Dr., 63691 Ranstadt (DE)
(74) Vertreter: Wolf, Günter, Dipl.-Ing.

(56) Entgegenhaltungen:
- EP-A- 0 818 390
- US-A- 4 008 401
- US-A- 4 193 204

## Beschreibung

Die Erfindung betrifft eine Vorrichtung zum Entkeimen von Packstoffbahnen, bestehend aus Einrichtungen zur Erzeugung von UV-Strahlen und aus Führungsmitteln für die Vorbeiförderung der Packstoffbahn am zuförderseitig in einem Steriltunnel angeordneten UV-Strahlenerzeuger.

Die Verwendung von UV-Strahlern zur Keimreduktion von Packstoffbahnen ist zum beispiel aus EP 0 818 390 bekannt, die entsprechend bestrahlt, entkeimt und im Steriltunnel weitergeführt die sich anschließenden Ausform-,Füll- und Schließstationen von Verpackungsmaschinen durchlaufen, an denen die Vorrichtungen zum Entkeimen die Einlaufstationen darstellen.
Bisherige Entkeimungssysteme auf UV-Basis arbeiten mit sogenannten, linear längs der Packstoffbahn angeordneten Strahlerkassetten, wobei zwecks Verbesserung der Strahlungsausnutzung die rückwärtige Seite der den bzw. die Strahler aufnehmenden Gehäuse bzw. Kassetten verspiegelt, d.h., reflektorartig ausgebildet sein muß. Um die erforderliche Strahlungsdosis zu erreichen, müssen ferner entweder sehr breite bzw. lange Sonderkassetten verwendet werden, oder es müssen mehrere Kassetten raumbeanspruchend längs der Packstoffbahnförderstrecke aneinandergereiht werden.
Aufgrund der in der Regel auf ca. 2000 h begrenzten Lebensdauer solcher UV-Strahler treten hohe Kosten für den Ersatz auf, wobei insbesondere auch die Einbau-, Wartungs- und Reinigungskosten und die damit verbundenen Stillstandszeiten derartige Systeme zu Buche schlagen.
Herkömmliche UV-Strahler führen außerdem zu einer nicht ünerheblichen Ozonbildung und können darüber hinaus den Packstoff negativ beeinflussen (Schadstoffmigration).
Wassergekühlte Strahlersysteme, die auch bekannt sind, bergen die Gefahr der Kondensatbildung bei Taupunktunterschreitung, was zur direkten Schwächung der UV-Leistungsabgabe auf den Packstoff infolge Kondensatbildung führen kann und außerdem zu indirekten Leistungsabgabeminderung infolge korrosiver Auswirkungen auf die Reflektorflächen.

Der Erfindung liegt die Aufgabe zugrunde, auf UV-Strahlerbasis eine Vorrichtung zum Entkeimen von Packstoffbahnen zu schaffen, die ohne Strahlungsreflektoren auskommt, trotz relativ großer Bestrahlungsfläche der Packstoffbahn wenig Raum beansprucht und damit auch gut abschirmbar ist, die eine gleichmäßige Bestrahlungsintensität bei hoher Leistungsdichte der vorbeilaufenden Packstoffbahn gewährleistet und die schließlich auch auf denkbar einfache Weise eine kassetten- bzw. gehäusefreie Zuordnung des "nackten" Strahlers zur zu bestrahlenden Packstoffbahn zuläßt und damit auch eine einfache und schnelle Auswechselbarkeit verbrauchter Strahler.

Diese Aufgabe ist mit einer Vorrichtung der eingangs genannten Art nach der Erfindung dadurch gelöst, daß der stabförmig ausgebildete UV-Strahlenerzeuger zentral zwischen mit gleichem radialen Abstand um den UV-Strahlenerzeuger angeordneten Führungsmitteln angeordnet ist, wobei der von diesen Mitteln definierte, kreisförmigen Packstoffbahnförderstrecke zu-und/oder auslaufseitig Packstoffbahnumlenkelemente zugeordnet sind, die die Packstoffbahn in die kreisförmige Förderstrecke ein- und/oder aus dieser herausleiten.
Vorteilhafte Weiterbildungen dieser Lösung ergeben sich nach den Unteransprüchen.

Mit dieser erfindungsgemäßen Lösung sind die gestellten Forderungen erfüllt. Die "und/oder"-Alternativen resultieren dabei, was noch näher erläutert wird, lediglich aus unterschiedlichen Möglichkeiten der Packstoffbahnzu- bzw. -einleitung in bzw. aus der kreisförmigen Bestrahlungsstrecke, auf die der zentral angeordnete, montagegünstig nur an einer Seite gehaltene bzw. gelagerte UV-Strahler ohne irgendwelche Reflektoren seine Strahlung allseitig mit voller und gleichmäßiger Leistungsdichte radial abgibt.

Diese erfindungsgemäße Lösung zieht grundsätzlich zunächst die Bestrahlung nur einer, d.h., der befüllseitigen Fläche der Packstoffbahn in Betracht, was letztlich darauf beruht, daß Packstoffbahnen auch in sterilen Halbtunneln in der sich anschließenden Verpackungsmaschine weitergefördert und verarbeitet werden. Da jedoch Verpackungsmaschinen mit sterilen Volltunneln konstruktiv weniger aufwendig auszubilden sind, also mit Steriltunneln, die die durchlaufende Packstoffbahn voll umfassen, besteht eine vorteilhafte und bevorzugte Ausführungs- bzw. Weiterbildungsform der Vorrichtung darin, daß der kreisförmigen Förder- bzw. Bestrahlungstrecke gegenüber eine weitere kreisförmige Förderstrecke zugeordnet ist, wobei die eine Förderstrecke mit Einleit- und die andere Förderstrecke mit Ausleitumlenkelementen versehen ist. Mit anderen Worten stellt dies gewissermaßen eine S-förmige Führung der beidflächig zu entkeimenden Packstoffbahn dar, wobei die aus der ersten Förderstrecke herauslaufende Packstoffbahn mit ihrer noch nicht entkeimten Fläche, nunmehr gegen den dortigen Strahler gewandt, in die unmittelbar benachbarte Förderstrecke einläuft, in der der dort ebenfalls zentral angeordnete UV-Strahler auf die noch nicht entkeimte Fläche der Packstoffbahn einwirkt.

Abgesehen von dieser unmittelbar benachbarten Zuordnung zweier solcher kreisförmigen, von der Packstoffbahn nacheinander zu durchlaufenden Förder- bzw. Bestrahlungsstrecken besteht ohne weiteres, was ebenfalls noch näher erläutert wird, die Möglichkeit, zwei oder mehr solcher Förderstreckenpaarungen hintereinander anzuordnen, wenn dies, vom gewünschten Entkeimungsgrad her gesehen, in Betracht zu ziehen ist.

Was die die kreisförmige Führung der Packstoffbahn definierenden Führungsmittel betrifft, so können diese aus stabförmigen Rollkörpern gebildet sein, es ist aber auch möglich, diese Führungsmittel in Form eines Zylinders aus UV-durchlässigem Material auszubilden.

Die erfindungsgemäße Vorrichtung wird nachfolgend an Hand der zeichnerischen Darstellung von Ausführungsbeispielen näher erläutert.

Es zeigt
- Fig.1: das Prinzip der kreisförmigen Führung der Packstoffbahn innerhalb eines Sterilgehäuses um einen zentral angeordneten UV-Strahler;
- Fig.2: eine der Fig.1 entsprechende Darstellung mit anderer Ausführungsform der Führungselemente;
- Fig.3: schematisch eine kombinierte Zusammenschaltung der Vorrichtungen nach den Fig.1,2;
- Fig.4: schematisch eine Draufsicht auf die Ausführungsform nach Fig.3;
- Fig.5: unter A bis E ebenfalls schematisch und ohne das Sterilgehäuse unterschiedliche Zu- und Auslaufführungen der Packstoffbahn zu und aus den kreisförmigen Förder- bzw. Bestrahlungsstrecken und
- Fig.6: schematisch die erfindungsgemäße Vorrichtung in Verbindung und Zuordnung zu einer nur umrißartig angedeuteten und nur als Beispiel zu verstehenden Verpakkungsmaschine.

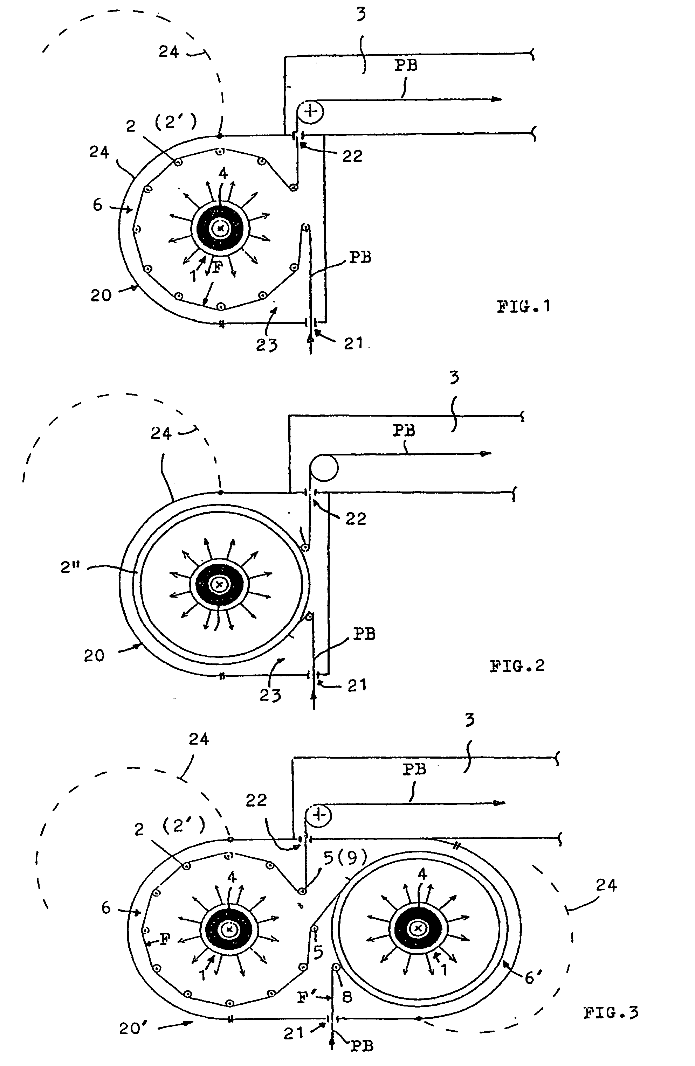

Die erfindungsgemäße Vorrichtung zum Entkeimen von Packstoffbahnen PB besteht aus Einrichtungen 1 zur Erzeugung von UV-Strahlen und aus Führungselementen 2 für die Vorbeiförderung der Packstoffbahn PB an einem UV-Strahler 4. Die Führungselemente 2 und der UV-Strahler 4 sind in einem Sterilgehäuse 20 angeordnet, das mit Ein- und Auslaßöffnungen 21,22 versehen ist und das unter leichtem Überdruck eines gasförmigen Sterilmittels bspw. Sterilluft steht, um einen Keimzutritt von außen zu verhindern.
Für eine solche Vorrichtung ist nun wesentlich, daß der stabförmig ausgebildete UV-Strahler 4 zentral zwischen den mit gleichem radialen Abstand um den UV-Strahler 4 angeordneten Führungselementen 2 angeordnet ist. Der von den Führungselementen 2 definierten, kreisförmigen Packstoffbahnförderstrecke 6 sind dabei am Ein-und/oder Auslauf Packstoffbahnumlenkelemente 5 zugeordnet, die die Packstoffbahn PB in die kreisförmige Förderstrecke 6 ein- und aus dieser herausleiten.
Beim Ausführungsbeispiel nach Fig.1 sind die Führungselemente 2 aus stabförmigen, in den Seitenwänden 23 des Sterilgehäuses 20 drehbar gelagerten, durchmesserkleinen Walzen 2' gebildet und beim Ausführungsbeispiel nach Fig.2 aus einem Zylinder 2" aus UV-durchlässigem Material. Dieser Zylinder 2" kann aber muß nicht drehbar gelagert sein.
Bei den beiden Vorrichtungen nach den Fig.1,2 wird, wie ersichtlich nur eine Seite F der Packstoffbahn PB bestrahlt, und zwar die, die später in der Verpackungsmaschine mit dem Füllgut in Berührung kommt.

Das Sterilgehäuse 20 ist vorteilhaft mit einem zu öffnenden Wandungsteil 24 versehen, der in Öffnungsstellung gestrichelt in Fig.1 mit dargestellt ist und auch in Fig.3, und zwar dort auf beiden Seiten des Gehäuses 20'. Bei geöffneten Wandungsteilen 24 kann die Packstoffbahn PB problemlos von Hand um die Führungselemente 2 gelegt und dann bis in die sich anschließende Verpackungsmaschine gezogen werden. Beim Wandungsteil 24 kann es sich auch entgegen der Darstellung um einen einfachen Deckel handeln, der vom Gehäuse 20 abnehmbar ist.
Beim Ausführungsbeispiel nach Fig.3 sind die beiden Vorrichtungen nach den Fig.1,2 miteinander kombiniert, um beide Seiten F,F' der Packstoffbahn bestrahlen zu können, was keiner besonderen Erläuterung bedarf, da sich dies ohne weiteres aus der Packstoffbahnführung und den angegebenen Pfeilen ergibt. Es sei darauf hingewiesen, daß eine solche paarweise Zuordnung von Vorrichtungen in einem gemeinsamen Gehäuse 20' auch gebildet werden kann durch Verwendung zweier Vorrichtungen gemäß Fig.1 oder gemäß Fig.2.

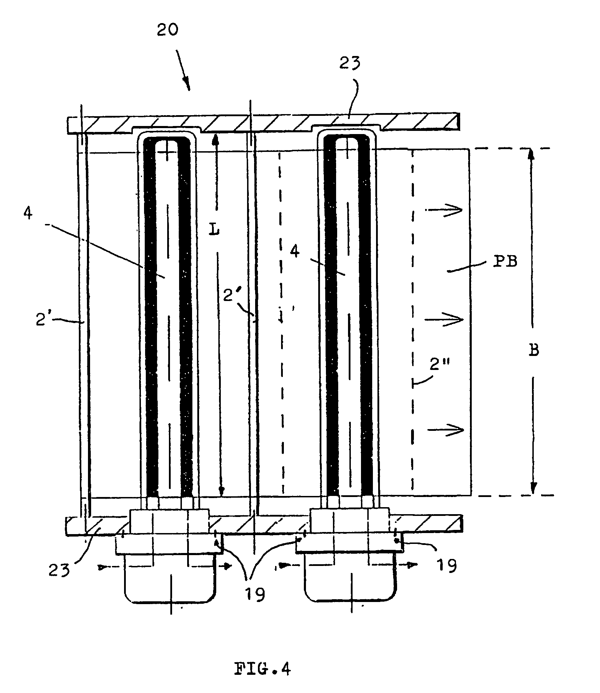

Fig.4 zeigt schematisch einen Schnitt durch das Ausführungsbeispiel nach Fig.3, der auch die stabförmige Gestalt der UV-Strahler 4 deutlich macht, deren Länge L im wesentlichen der Breite B der Packstoffbahn PB entspricht.
Diese Strahler 4, bei denen es sich auch um kühlbare Strahler handeln kann, sind in einer der Seitenwände 23 des Sterilgehäuses 20 in geeigneter Weise angeordnet und austauschbar mit Halteelementen 19 fixiert, können also leicht eingebaut und nach Ablauf ihrer Lebensdauer auch leicht ausgetauscht werden. Stromanschlüsse sind nicht dargestellt, da selbstverständlich.

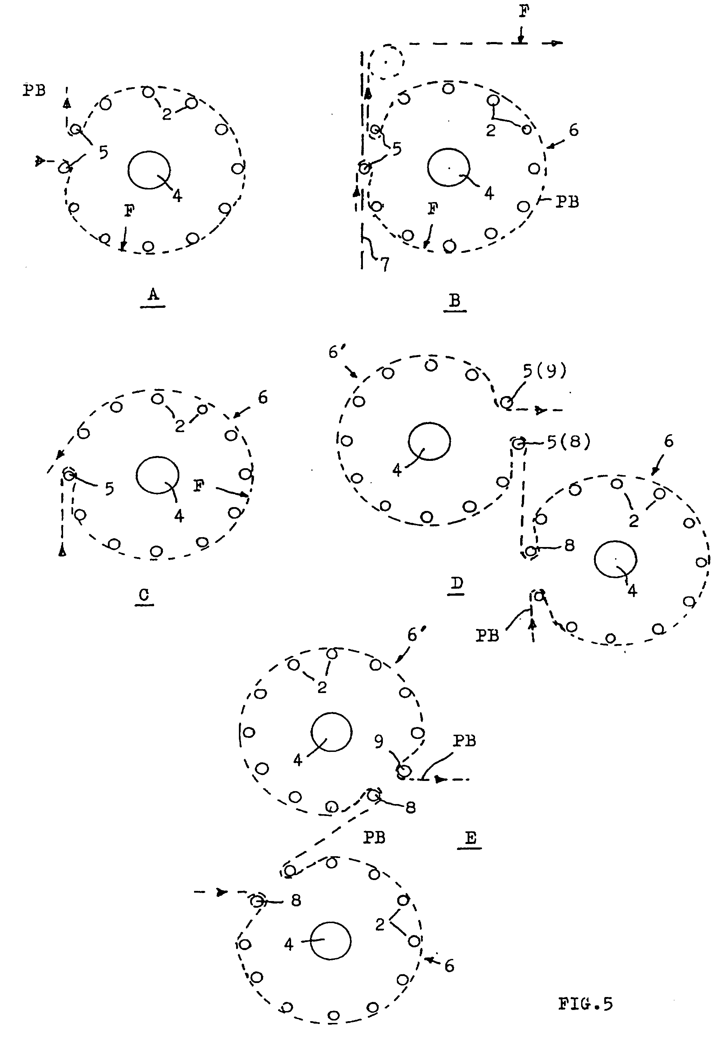

Für die Zuführung der Packstoffbahn in die erfindungsgemäße Vorrichtung und deren Weiterleitung aus der Vorrichtung zur sich anschließenden Verpackungsmaschine bestehen die unterschiedlichsten Möglichkeiten, von denen einige in der Fig. 5 dargestellt und dort mit A bis E bezeichnet sind.
Von diesen Möglichkeiten wird jedoch insbesondere die gemäß Fig. 3B in Betracht gezogen, bei der die Packstoffbahnumlenkelemente 5 im wesentlichen längs einer die kreisförmige Förderstrecke 6 tangierenden Geraden 7 angeordnet sind. Letztlich richtet sich dies aber nach der einlaufseitigen Gestaltung der der Vorrichtung nachgeschalteten Verpackungsmaschine.

Bei den Packstoffbahnführungsbeispielen nach den Fig.1,2 und 5 A bis C wird die durch die im wesentlichen immer kreisförmige Förderstrecke 6 laufende Packstoffbahn PB nur einseitig von der UV-Strahlung erfaßt, welche bestrahlte Fläche F dann in der Verpackungsmaschine die füllgutseitige Fläche des Packstoffes bildet, d.h., die, die entkeimt sein muß.

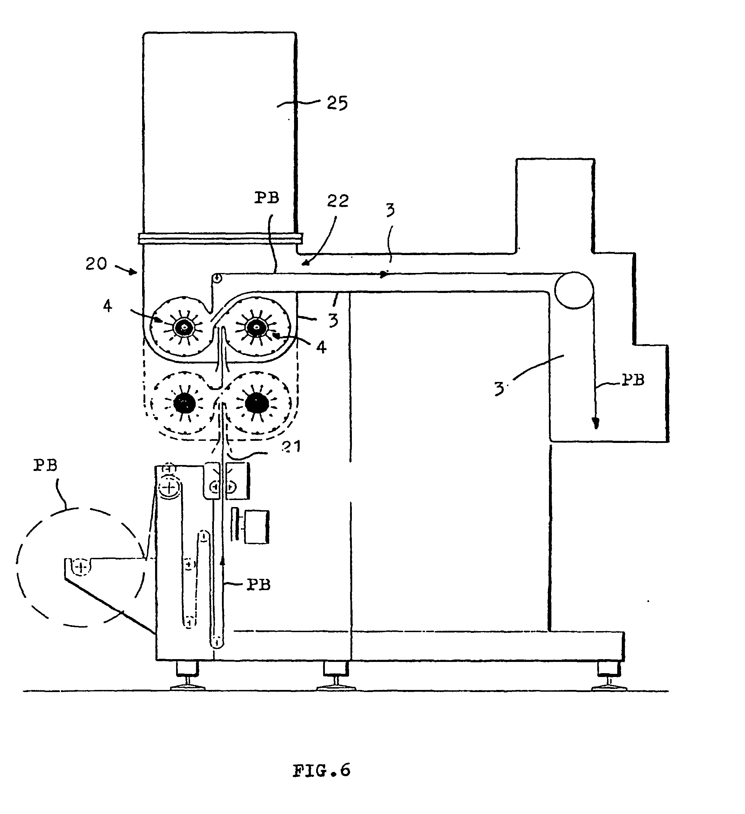

Aus den einleitend genannten Gründen wird jedoch eine beidseitige Bestrahlung und Entkeimung der Packsatoffbahn PB bevorzugt. Dafür ist unter Verweis auf Fig.3,4 und 5 D,E der kreisförmigen Förderstrecke 6 eine weitere, kreisförmige Förderstrecke 6' zugeordnet, wobei die eine Förderstrecke 6 mit Einleitelementen 8 und die andere Förderstrecke 6' mit Ausleitumlenkelementen 9 versehen ist. Unter "zugeordnet" sind dabei Zuordnungen der Förderstrecken 6,6', wie bspw. in diesen Figuren dargestellt, zu verstehen.
Im Bedarfsfall können solche Zuordnungspaarungen von Förderstrecken 6,6' längs der Packstoffbahnförderstrecke zur jeweiliegen Verpackungsmaschine auch mehrfach vorgesehen werden, wovon das Beispiel einer Doppelpaarung in Fig.6 dargestellt ist, die auch gleichzeitig ein Zuordnungsbeispiel der Vorrichtung zum sterilen Volltunnel 3 einer ansonsten nur schematisch dargestellten Verpackungsmaschine verdeutlicht, bei der es sich hier um eine Maschine zur Herstellung von sogenannten Schlauchbeuteln handelt. Bei diesem Ausführungsbeispiel ist über dem Gehäuse 20 der erfindungsgemäßen Vorrichtung zur Entkeimung ein Sterillufterzeuger 25 angeordnet, der also nicht nur die Vorrichtung mit leichtem Überdruck mit Sterilluft versorgt, sondern auch den Volltunnel 3 der Verpackungsmaschine, der, wie dargestellt, direkt an die Auslauföffnung 22 des Sterilgehäuses 20 angeschlossen ist.

Nicht besonders dargestellt ist eine Tiefziehverpackungsmaschine, deren Förderstrecke und damit die Packstoffbahn nur einseitig oberhalb mit einem sogenannten Halbtunnel steril gehalten wird. Für eine solche Maschine kommen Vorrichtungen nach den Fig.1,2 in Betråcht, also mit Packstoffbahnführungen gemäß der Fig. 5 A bis C, da bei Halbtunneln die nicht entkeimte Seite der Packstoffbahn PB nicht in das Innere des Halbtunnels weist.

Besonderer Förderelemente für die Packstoffbahn PB in der Vorrichtung nach der Erfindung bedarf es nicht, da die zu entkeimende Packstoffbahn PB von den Förderelementen in der angeschlossenen Verpackungsmaschine mit deren Arbeitstakt durch die Vorrichtung gezogen wird.

## Patentansprüche

1. Vorrichtung zum Entkeimen von Packstoffbahnen, bestehend aus Einrichtungen (1) zur Erzeugung von UV-Strahlen und aus Führungsmitteln (2) für die Vorbeiförderung der Packstoffbahn (PB) am zuförderseitig in einem Steriltunnel (3) angeordneten UV-Strahlenerzeuger (4),
**dadurch gekennzeichnet,**
**daß** der stabförmig ausgebildete UV-Strahlenerzeuger (4) zentral zwischen mit gleichem radialen Abstand um den UV-Strahlenerzeuger (4) angeordneten Führungsmitteln (2) angeordnet ist, wobei der von diesen definierten, kreisförmigen Packstoffbahnförderstrecke (6) zu- und/oder auslaufseitig Packstoffbahnumlenkelemente (5) zugeordnet sind, die die Packstoffbahn (PB) in die kreisförmige Förderstrecke (6) ein- und/oder aus dieser herausleiten.

2. Vorrichtung nach Anspruch 1,
**dadurch gekennzeichnet,**
**daß** die Packstoffbahnumlenkelemente (5) im wesentlichen längs einer die kreisförmige Förderstrecke (6) tangierenden Geraden (7) angeordnet sind.

3. Vorrichtung nach Anspruch 1 oder 2,
**dadurch gekennzeichnet,**
**daß** der kreisförmigen Förderstrecke (6) gegenüber eine weitere kreisförmige Förderstrecke (6') zugeordnet ist, wobei die eine Förderstrecke (6) mit Einleit-(8) und die andere Förderstrecke (6') mit Ausleitumlenkelementen(9) versehen ist.

4. Vorrichtung nach einem der Ansprüche 1 bis 3,
**dadurch gekennzeichnet,**
**daß** die um den UV-Strahlenerzeuger (4) kreisförmig angeordneten Führungsmittel (2) aus stabförmigen Rollkörpern (2') gebildet sind.

5. Vorrichtung nach einem der Ansprüche 1 bis 3,
**dadurch gekennzeichnet,**
**daß** die um den UV-Strahlenerzeuger (4) kreisförmig angeordneten Führungsmittel (2) zylindrisch ausgebildet und aus UV-durchlässigem Material gebildet sind.

6. Vorrichtung nach einem der Ansprüche 1 bis 5,
**dadurch gekennzeichnet,**
**daß** die kreisförmige Förderstrecke (6,6') in einem mindestens teilweise formangepaßten Gehäuse (20) mit Ein- und Auslauföffnungen (21,22) angeordnet ist.

7. Vorrichtung nach Anspruch 6,
**dadurch gekennzeichnet,**
**daß** das Gehäuse (20) mit einem zu öffnenden Wandungsteil (24) versehen ist.

8. Vorrichtung nach einem der Ansprüche 6 oder 7,
**dadurch gekennzeichnet,**
**daß** die Packstoffbahnführungsmittel (2) zwischen Seitenwänden (23) des Gehäuses (20) angeordnet sind.

9. Vorrichtung nach Anspruch 8,
**dadurch gekennzeichnet,**
**daß** die UV-Strahlenerzeuger (4) einseitig an einer der Seitenwände (23) einsteck- und lösbar angeordnet sind.

10. Vorrichtung nach einem der Ansprüche 6 bis 9, **dadurch gekennzeichnet,**
**daß** die Auslauföffnung (22) die Einlauföffnung eines Steriltunnels (3) bildet.

## Claims

1. A device for degerming continuous strips of packaging materials, including means (1) for generating UV rays and guidance means (2) for conveying the continuous strip of packaging material (PB) in front of the means for generating UV rays (4) that is arranged downstream in a sterile tunnel (3),
**characterised in that**
the rod-shaped means (4) for generating UV-rays is disposed centrally between guidance means (2) that are arranged radially equidistantly about the means (4) for generating UV rays, wherein redirecting elements (5) on the infeed and/or the outfeed sides are assigned to the circular conveyor line (6) for the packaging materials that is defined thereby and guide the packaging material strip (PB) into and/or out of the circular conveyor line (6).

2. The device according to claim 1,
**characterised in that**
the packaging material strip redirecting elements (5) are arranged essentially along the length of a straight line (7) tangent upon the circular conveyor line (6).

3. The device according to claim 1 or 2,
**characterised in that**
the circular conveyor line (6) is arranged opposite a second circular conveyor line (6'), first circular conveyor line (6) being provided with infeed guidance elements (8), and the second circular conveyor line (6') being provided with outfeed guidance elements (9).

4. The device according to any of claims 1 to 3,
**characterised in that**
the guidance means (2) arranged in a circular pattern about the means (4) for generating UV-rays are constructed from rod-shaped rolling bodies (2').

5. The device according to any of claims 1 to 3,
**characterised in that**
the guidance means (2) arranged in a circular pattern about the means (4) for generating UV-rays are cylindrical in shape and made from a material that is impermeable to UV rays.

6. The device according to any of claims 1 to 5,
**characterised in that**
the circular conveyor line (6, 6') is arranged in an at least partially shape-conformant housing (20) having infeed and outfeed openings (21, 22).

7. The device according to claim 6,
**characterised in that**
the housing (20) is furnished with a partitioning element (24) that must be opened.

8. The device according to either of claims 6 or 7,
**characterised in that**
the guidance means (2) for the strip of packaging material are arranged between lateral walls (23) of the housing (20).

9. The device according to claim 8,
**characterised in that**
the means for generating UV rays (4) are arranged on one side of one of the lateral walls (23) in such manner that they can be installed and detached.

10. The device according to any of claims 6 to 9,
**characterised in that**
the outfeed opening (22) forms the infeed opening of a sterile tunnel (3).

## Revendications

1. Dispositif de stérilisation de bandes de matériau d'emballage, consistant en installations (1) servant à générer des rayons UV et en moyens de guidage (2) servant à faire passer la bande de matériau d'emballage (PB) devant le générateur de rayons UV (4) disposé côté acheminement dans un tunnel stérile (3),
**caractérisé en ce que**
le générateur de rayons UV (4) réalisé en forme de barre (4) est disposé au centre entre des moyens de guidage (2) disposés à la même distance radiale autour du générateur de rayons UV (4), étant associés au segment de transport (6) de la bande de matériau d'emballage circulaire défini par ceux-ci, côté entrée et/ou sortie, des éléments de déviation de la bande de matériau d'emballage (5) qui guident la bande de matériau d'emballage (PB) dans le segment de transport circulaire (6) et/ou hors de celui-ci.

2. Dispositif selon la revendication 1,
**caractérisé en ce que**
les éléments de déviation de la bande de matériau d'emballage (5) sont disposés sensiblement le long d'une droite (7) tangente au segment de transport circulaire (6).

3. Dispositif selon la revendication 1 ou 2,
**caractérisé en ce**
**qu'**est associé au segment de transport circulaire (6), en face, un autre segment de transport circulaire (6'), un segment de transport (6) étant équipé d'éléments de déviation d'introduction (8) et l'autre segment de transport (6') d'éléments de déviation d'évacuation (9).

4. Dispositif selon l'une quelconque des revendications 1 à 3,
**caractérisé en ce que**
les moyens de guidage (2) disposés en forme circulaire autour du générateur de rayons UV (4) sont formés de corps roulants en forme de barres (2').

5. Dispositif selon l'une quelconque des revendications 1 à 3,
**caractérisé en ce que**
les moyens de guidage (2) disposés en forme circulaire autour du générateur de rayons UV (4) sont conçus sous forme cylindrique et sont formés d'un matériau perméable aux UV.

6. Dispositif selon l'une quelconque des revendications 1 à 5,
**caractérisé en ce que**
le segment de transport circulaire (6, 6') est disposé dans une enceinte (20) adaptée au moins partiellement en forme et comportant des orifices d'entrée et de sortie (21, 22).

7. Dispositif selon la revendication 6,
**caractérisé en ce que**
l'enceinte (20) est équipée d'une pièce de paroi ouvrante (24).

8. Dispositif selon l'une quelconque des revendications 6 ou 7,
**caractérisé en ce que**
les moyens de guidage de la bande de matériau d'emballage (2) sont disposés entre les parois latérales (23) de l'enceinte (20).

9. Dispositif selon la revendication 8,
**caractérisé en ce que**
le générateur de rayons UV (4) est disposé d'un côté de manière insérable et détachable sur une des parois latérales (23).

10. Dispositif selon l'une quelconque des revendications 6 à 9,
**caractérisé en ce que**
l'orifice de sortie (22) constitue l'orifice d'entrée d'un tunnel stérile (3).
